# EUROPEAN PATENT APPLICATION

(11) **EP 1 180 355 A1**
(43) Date of publication of application: **20.02.2002**
(21) Application number: 01115686.6
(22) Date of filing: 04.07.2001
(51) Int. Cl.: A61K 6/083

(54) **Glass ionomer-based fluoride applying material**

(30) Priority: 17.08.2000 JP 2000247731
(71) Applicant: GC Corporation, Itabashi-ku, Tokyo 174 (JP)
(72) Inventor: Kobayashi, Keizo, Itabashi-ku, Tokyo (JP); Hirota, Kazuo, Itabashi-ku, Tokyo (JP)
(74) Representative: Müller-Boré & Partner Patentanwälte

(57) **Abstract**

A glass ionomer-based fluoride applying material that is able to release 200 µg/cm² or more, and preferably 300 µg/cm² or more of a fluoride ion until 30 days after immersing a set material thereof in distilled water kept at 37 °C, is provided, which contains a fluoroaluminosilicate glass powder, a polycarboxylic acid and water as major components, the fluoroaluminosilicate glass powder accounting for 5 to. 70 % by weight of the whole, preferably containing, as constitutional elements, 10 to 20 % by weight of Si, 10 to 20 % by weight of Al, 1 to 20 % weight of F, and 5 to 30 % by weight of Sr, and additionally, at least one of Na, K, Mg and Ca, and preferably containing Na in an amount of 3 to 15 % by weight.

## Description

The present invention relates to a glass ionomer-based fluoride applying material and more specifically, to a glass ionomer-based fluoride applying material that is used for accelerating a remineralization action of a tooth.

Although enamel that covers an outer layer of a tooth is the hardest tissue in a human body, it is a tissue exhibiting an acute reaction with acids. Since the enamel is almost constructed of a crystal of hydroxyapatite (Ca₁₀(PO₄)₆(OH)₂), the hydroxyl groups thereof are liable to react with an acid. Thus, the following matter is known. That is, usually, in the oral cavity, elution of a phosphate ion and a calcium ion (demineralization) is in an equilibrium state with crystallization into calcium phosphate and hydroxyapatite (remineralization). Accordingly, during dental caries, bacteria contained in a plaque, such as *Streptococcus mutans*, decompose sucrose to produce an organic acid composed mainly of lactic acid, and the produced acid lowers the pH, and the enamel releases the calcium ion and the like. Thus, the equilibrium state deviates into the elution side so that the demineralization of the enamel is accelerated, thereby causing dental caries.

On the other hand, it is known that at the initial stage of the dental caries, when a fluoride ion is applied to a white spot generated by demineralization on usually transparent enamel, the growth rate of the hydroxyapatite crystal is made effectively fast via fluoroapatite to accelerate the mineralization, whereby the white spot by the demineralization disappears. For this reason, it is widely employed at present to prevent the dental caries by accelerating the remineralization of the enamel using a composition for releasing a fluoride ion.

In general, examples of application of a fluoride ion, which is aimed at the prevention of dental caries, include a whole-body application in which about 1 ppm of fluoride is contained in tap water and a local application in which a fluoride is applied only in an oral cavity, for the purpose of forming enamel resisting against the dental caries. Further, as the local application, are employed a fluoride-applying method in which an aqueous solution containing a fluoride is applied directly to a tooth surface and a fluoride mouth-rinsing method in which the oral cavity is rinsed with an aqueous solution containing a low concentration of a fluoride.

The fluoride-applying method is a method in which an 8 % stannous fluoride solution is applied once or twice a year. However, the stannous fluoride solution tastes puckery and is instable such that the effect is liable to be lost. Thus, it is inconvenient in handling. Further, the tooth surface was possibly colored brown.

As the fluoride mouth-rinsing method, is generally employed a method in which the oral cavity is rinsed with, for example, an aqueous sodium fluoride solution as a rinsing agent, in an amount of about 5 mℓ for 30 seconds to one minute. However, since the fluoride ion is merely fed occasionally to the whole of the oral cavity according to this method, the fluoride ion could not be surely applied to the white spot of the enamel.

Thus, the present inventors paid attention to a dental glass ionomer cement having not only fluoride ion-releasing properties but also adhesive properties to the tooth surface. When the dental glass ionomer cement as an oral composition for releasing a fluoride ion is applied to the white spot of the tooth surface and then set, a set material of the cement can remain on the white spot of the enamel over a long period of time due to dentinal adhesive properties as a characteristic feature of the dental glass ionomer cement composition. Then, the inventors also paid attention to the matter that when this dental glass ionomer cement is adhered to the white spot, it can be made a direct supply source for a fluoride ion, and made investigations on the application of the conventional art dental glass ionomer cement as a fluoride ion-releasing glass ionomer-based fluoride applying material.

The dental glass ionomer cement composition is aimed originally at filling remedy of a cavity or cementing of a prosthesis but not aimed primarily at release of a fluoride ion. Accordingly, with respect to generally employed dental glass ionomer cement compositions, in the case where a set material obtained by charging a dental glass ionomer cement composition into each of two cylindrical holes (depth: 1 mm, diameter: 6 mm) provided on an acrylic resin-made mold is immersed in distilled water kept at 37 °C without being taken out from the acrylic resin-made mold, it had a fluoride ion-releasing rate as specified below of about 160 µg/cm², and at most 180 µg/cm² for products releasing the largest amount of the fluoride ion, until 30 days after immersing. Here, the fluoride ion-releasing rate is a value obtained by dividing the sum of the released fluoride ions by an area [3 mm (radius) × 3 mm (radius) × π (circular constant) × 2] of the set material coming into contact with distilled water. Such fluoride ion-releasingrate was insufficient for remineralizing the white spot on the enamel as a glass ionomer-based fluoride applying material for preventing the dental caries from occurrence.

The invention is aimed to provide a glass ionomer-based fluoride applying material, which is able to continuously supply a fluoride ion directly to a white spot of enamel over a longer period of time and has a more fluoride ion-releasing rate, specifically 200 µg/cm² or more, and preferably 300 µg/cm² or more, as compared with the conventional art rinsing agents used for fluoride mouth-rinsing.

In order to achieve the above-described aim, we, the present inventors made extensive and intensive investigations. As a result, it has been astonishingly found that when a proportion of a fluoroaluminosilicate glass powder in a dental glass ionomer cement composition is 5 to 70 % by weight of the whole of the composition, a larger amount of a fluoride ion can be released, as compared with the conventional art glass ionomer cement compositions; and that when the fluoroaluminosilicate glass powder is compounded in a specified proportion, a much larger amount of the fluoride ion can be released, leading to accomplishment of the glass ionomer-based fluoride applying material according to the present invention.

It is not the case that a major part of the fluoride ion of the dental glass ionomer cement composition is released from the unreacted fluoroaluminosilicate glass powder or a fluoride. But, when the fluoroaluminosilicate glass powder reacts with a polycarboxylic acid via water, a large amount of a fluoride ion is released. In this regard, since the conventional art dental glass ionomer cement compositions are aimed at dentinal filling remedy, in order to enhance the strength of the set material, it was necessary to compound a larger amount of the fluoroaluminosilicate glass powder than that of the polycarboxylic acid. Accordingly, it is considered that the fluoride ion is hardly released.

Specifically, the present invention is concerned with a glass ionomer-based fluoride applying material comprising a fluoroaluminosilicate glass powder, a polycarboxylic acid and water as major components, wherein the fluoroaluminosilicate glass powder accounts for 5 to 70 % by weight of the whole, and when a set material thereof is immersed in distilled water kept at 37 °C, a fluoride ion of 200 µg/cm² or more is released until 30 days after immersing.

Further, it has been found that in the case where a set material made of the glass ionomer-based fluoride applying material that is constructed of 10 to 50 % by. weight of the fluoroaluminosilicate glass powder, 20 to 60 % by weight of the polycarboxylic acid, and 30 to 70 % by weight of water is immersed in distilled water kept at 37 °C, a fluoride ion of 300 µg/cm² or more can be released until 30 days after immersing. It has also been found that the fluoroaluminosilicate glass powder is preferably comprised of, as constitutional elements, 10 to 20 % by weight of Si, 10 to 20 % by weight of Al, 1 to 20 % weight of F, and 5 to 30 % by weight of Sr, and additionally, at least one of Na, K, Mg and Ca in a total amount of 3 to 15 % by weight; and that Na is more preferable among Na, K, Mg and Ca.

A suitable amount of the fluoroaluminosilicate glass powder of the glass ionomer-based fluoride applying material according to the present invention is 5 to 70 % by weight of the whole. In order to obtain a more effect, it is preferred that the amount of the fluoroaluminosilicate glass powder that reacts with the polycarboxylic acid via water is increased, namely it ranges from 10 to 50 % by weight, and more preferably from 10 to 30 % by weight. When the amount of the fluoroaluminosilicate glass powder exceeds 70 % by weight, not only the amount of the released fluoride ion is not sufficient, but also the operability for applying the composition before set to a white spot of enamel is deteriorated. On the other hand, when the amount of the fluoroaluminosilicate glass powder is less than 5 % by weight, the physical properties of the set material are lowered, whereby the set material cannot remain on the enamel over a long period of time.

When the fluoroaluminosilicate glass powder that is used in the glass ionomer-based fluoride applying material according to the present invention is comprised of, as constitutional elements, 10 to 20 % by weight of Si, 10 to 20 % by weight of Al, 1 to 20 % weight of F, and 5 to 30 % by weight of Sr, and additionally, at least one of Na, K, Mg and Ca in a total amount of 3 to 15 % by weight, a larger amount of the fluoride ion can be released.

When the proportion of Si is less than 10 % by weight, it is difficult to prepare the glass, whereas when it exceeds 20 % by weight, the strength of the set material is low. When the proportion of Al is less than 10 % by weight, the strength of the set material is low, whereas when it exceeds 20 % by weight, it is difficult to prepare the glass. When the proportion of F is less than 1 % by weight, the amount of the released fluoride ion is low. On the other hand, even when the proportion of F exceeds 20 % by weight, not only the increase of the amount of the released fluoride ion is not attained, but also the set material is brittle so that it is inferior in durability. When the proportion of Sr is less than 5 % by weight, not only the setting time of the glass ionomer-based fluoride applying material is liable to be prolonged, but also it is difficult to prepare the glass. On the other hand, when the proportion of Sr exceeds 30 % by weight, the working time is too short, whereby the use and operation are difficult. Further, as at least one of Na, K, Ma and Ca is contained, though the amount of the released fluoride ion tends to increase, the physical properties of the set material are markedly lowered. Accordingly, the total amount of at least one of Na, K, Ma and Ca is 15 % by weight or less. When the total amount of at least one of Na, K, Ma and Ca is less than 3 % by weight, the effect for increasing the amount of the released fluoride ion is lowered. Of these elements, Na is the most effective. These Na, K, Ma and Ca elements are compounded during the melting step of glass, and in general, known materials that are usually and widely used in oxides, fluorides, phosphates and the like can be used. Moreover, as the case may be, a part of these elements can be added to the fluoroaluminosilicate glass powder not as glass components but as fluorides. Suitable examples include sodium fluoride, potassium fluoride, calcium fluoride, and strontium fluoride.

With respect to the fluoroaluminosilicate glass powder used for the glass ionomer-based fluoride applying material according to the present invention, its particle size may influence the amount of the released fluoride ion. The smaller the particle size of the fluoroaluminosilicate glass powder, the more improved the fluoride ion releasing properties. Preferably, a mean particle size of the fluoroaluminosilicate glass powder is in the range from 0.5 µm to 25 µm. Although the increase of the amount of the released fluoride ion is found as the particle size is smaller, when the mean particle size exceeds 25 µm or is smaller than 0.5 µm, the operability of the composition before setting is deteriorated. More preferably, the mean particle size is in the range from 0.5 µm to 10 µm.

As the polycarboxylic acid that is used for the glass ionomer-based fluoride applying material according to the present invention, known polycarboxylic acids and polyphosphonic acids that are used in the conventional art dental glass ionomer cement compositions can be used. Examples include polyacrylic acid, polymaleic acid, vinyl phosphonic acid, and copolymers thereof. A compounding amount of the polycarboxylic acid is preferably 20 to 60 % by weight of the whole of the applying material.

In the glass ionomer-based fluoride applying material according to the present invention, since a coagulation reaction (neutralization) between the fluoroaluminosilicate glass powder and the polycarboxylic acid is carried out via water, the presence of water is indispensable. A compounding amount of water is preferably 30 to 70 % by weight of the whole of the applying material.

In the glass ionomer-based fluoride applying material according to the present invention, when the fluoroaluminosilicate glass powder, the polycarboxylic acid and water are mixed in a predetermined proportion, and the mixture is applied to a white spot generated on a tooth surface, between teeth, or in a fissure using a brush, etc., a set material of the fluoride applying material can be kept as a fluoride ion-supply source on the white spot. Further, the glass ionomer-based fluoride applying material according to the present invention is also effective as a material to be applied to a cavity bottom portion before filling with a restorative when cavity has been formed. In this case, since the content of the glass powder is low, it is easy to apply it to the cavity bottom portion, and a large amount of the fluoride ion is released. Accordingly, it is effective for preventing secondary dental caries in the cavity bottom portion from occurrence. Example 1:

Forty-one grams of SiO₂, 23 g of Al₂O₃, 13 g of Ca₂(H₂PO₄)₂, 13 g of Al₂(HPO₄)₃, and 10 g of CaF₂ were thoroughly mixed and stirred in a mortar. The obtained batch was charged in a ceramics crucible, and the temperature was elevated to 1,100 °C at a temperature-elevation rate of about 7 °C/min. in an electric furnace. After holding for 5 hours, the melt was made to flow into water for quenching to obtain a glass. The obtained glass was ground in a ball mill to prepare a fluoroaluminosilicate glass powder A having a mean particle size of 2.32 µm. 0.4 g of this glass powder A was mixed with 1.5 g of a commercially available glass ionomer cement liquid (a trade name: Fuji I Liquid, made by GC Corporation). The mixture (a glass powder content: 21 % by weight) was measured for a fluoride ion-releasing rate from its set material. The results obtained are shown in Table 1.

### Measurement of fluoride ion-releasing rate:

The mixture before setting was charged into each of two cylindrical holes (depth: 1 mm, diameter: 6 mm) provided on an acrylic resin-made mold and brought into press contact with a glass-made plate. After allowing it to set, the glass-made plate was removed. The obtained set material was held in a chamber kept at a temperature of 37 °C and at a humidity of 100 % for one hour without being taken out from the acrylic resin-made mold. Subsequently, the obtained set material was immersed in 8 mℓ of distilled water and kept in a chamber at 37 °C without being taken out from the acrylic resin-made mold. After a lapse of 30 days, the resulting set material was discharged from the thermostat and rinsed with 2 mℓ of distilled water. The sum of a fluoride ion that had eluted into 10 mℓ of water including the rinsing water was measured using an ion meter provided with fluoride electrodes. The sum of the fluoride ion as measured was divided by an exposed surface area of the set material, to obtain the fluoride ion-releasing rate.

### Examples 2 and 3:

In Example 2, 1.0 g of the fluoroaluminosilicate glass powder A was mixed with 1.5 g of a commercially available glass ionomer cement liquid (a trade name: Fuji I Liquid, made by GC Corporation). The mixture (a glass powder content: 40 % by weight) was tested in the same manner as in Example 1. The results obtained are summarized and shown in Table 1.

In Example 3, the fluoroaluminosilicate glass powder A was mixed with a commercially available glass ionomer cement liquid (a trade name: Fuji I Liquid, made by GC Corporation) to prepare a mixture having a glass powder content of 63 % by weight, which was then tested in the same manner as in Example 1. The results obtained are summarized and shown in Table 1.

### Examples 4 to 6:

31 g of SiO₂, 23 g of Al₂O₃, 9 g of Na₂AlF₆, 2 g of Al₂(HPO₄)₃, 1 g of CaF₂, and 34 g of SrF₂ were thoroughly mixed and stirred in a mortar. The obtained batch was charged in a ceramics crucible, and the temperature was elevated to 1,350 °C at a temperature-elevation rate of about 7 °C/min. in an electric furnace. After holding for 3 hours, the melt was made to flow into water for quenching to obtain a glass. The obtained glass was ground in a ball mill to prepare a fluoroaluminosilicate glass powder B having a mean particle size of 2.11 µm. This fluoroaluminosilicate glass powder B was mixed with a commercially available glass ionomer cement liquid (a trade name: Fuji I Liquid, made by GC Corporation) such that the glass powder content was 13 % by weight (Example 4), 28 % by weight (Example 5) and 68 % by weight (Example 6), respectively. Then, each of the mixtures was tested in the same manner as in Example 1. The results obtained are summarized and shown in Table 1.

### Examples 7 to 9:

A fluoroaluminosilicate glass powder C having a composition comprising 35 g of SiO₂, 27 g of Al₂O₃, 14 g of Na₂AlF₆, 5 g of Al₂(HPO)₄)₃, 9 g of CaF₂ and 10 g of SrF₂ was prepared in the same procedures as in Example 1. This glass powder had a mean particle size of 1.80 µm. This glass powder was mixed with a commercially available glass ionomer cement liquid (a trade name Fuji IXGP Liquid, made by GC Corporation), and the mixture was tested in the same manner as in Example 1. The results obtained are summarized and shown in Table 1.

### Comparative Examples 1 to 4:

Each of the fluoroaluminosilicate glass powders A, B and C was mixed with a commercially available glass ionomer cement liquid (a trade name: Fuji I Liquid, made by GC Corporation) in a proportion such that the glass powder content fell outside of the present invention. Then, the amount of the released fluoride ion from the set material was measured in the same manner as in Example 1. The results obtained are summarized and shown in Table 1.

**Table 1**

| | Fluoroaluminosilicate glass powder | Amount of compounded fluoroaluminosilicate glass powder | Total fluoride ion-releasing rate after 30 days (µg/cm²) |
|---|---|---|---|
| Example 1 | Glass powder A | 21 % by weight | 490 |
| Example 2 | Ditto | 40 % by weight | 308 |
| Example 3 | Ditto | 63 % by weight | 210 |
| Example 4 | Glass powder B | 13 % by weight | 1332 |
| Example 5 | Ditto | 28 % by weight | 1187 |
| Example 6 | Ditto | 68 % by weight | 543 |
| Example 7 | Glass powder C | 9 % by weight | 1481 |
| Example 8 | Ditto | 18 % by weight | 1303 |
| Example 9 | Ditto | 27 % by weight | 1019 |
| Comparative Example 1 | Glass powder A | 75 % by weight | 159 |
| Comparative Example 2 | Glass powder B | 71 % by weight | 182 |
| Comparative Example 3 | Glass powder C | 80 % by weight | 103 |
| Comparative Example 4 | Glass powder A | 1 % by weight | Not set |

As has been described above in detail, in the glass ionomer-based fluoride applying material according to the present invention, the proportion of the fluoroaluminosilicate glass powder is 5 to 70 % by weight of the whole of the glass ionomer-based fluoride applying material, whereby a larger amount of a fluoride ion can be released, as compared with the conventional art dental glass ionomer cement composition. Further, when the proportion of the fluoroaluminosilicate glass powder is set up to a specific one, a much larger amount of the fluoride ion can be released. Accordingly, the present invention is a useful for remineralizing the white spot on the tooth.

While the invention has been described in detail and with reference to specific embodiments thereof, it will be apparent to one skilled in the art that various changes and modifications can be made therein without departing from the spirit and scope thereof.

## Claims

1. A glass ionomer-based fluoride applying material comprising a fluoroaluminosilicate glass powder, a polycarboxylic acid and water as major components, wherein the fluoroaluminosilicate glass powder accounts for 5 to 70 % by weight of the whole and, when a set material thereof is immersed in distilled water kept at 37 °C, a fluoride ion of 200 µg/cm² or more is released until 30 days after immersing.

2. The glass ionomer-based fluoride applying material as claimed in claim 1, wherein the fluoroaluminosilicate glass powder accounts for 10 to 50 % by weight, the polycarboxylic acid accounts for 20 to 60 % by weight, and water accounts for 30 to 70 % by weight, and, when a set material thereof is immersed in distilled water kept at 37 °C, a fluoride ion of 300 µg/cm² or more is released until 30 days after immersing.

3. The glass ionomer-based fluoride applying material as claimed in claim 1 or 2, wherein the fluoroaluminosilicate glass powder that is comprised of, as constitutional elements, 10 to 20 % by weight of Si, 10 to 20 % by weight of Al, 1 to 20 % weight of F, and from 5 to 30 % by weight of Sr, and additionally, at least one of Na, K, Mg and Ca in a total amount of 3 to 15 % by weight.

4. The glass ionomer-based fluoride applying material as claimed in claim 1 or 2, wherein the fluoroaluminosilicate glass powder that is comprised of, as constitutional elements, 10 to 20 % by weight of Si, 10 to 20 % by weight of Al, 1 to 20 % weight of F, and 5 to 30 % by weight of Sr, and additionally, Na in an amount of 3 to 15 % by weight.
